# EUROPEAN PATENT SPECIFICATION

(11) **EP 3 666 760 B1**
(45) Date of publication and mention of the grant of the patent: **26.07.2023**
(21) Application number: 19210282.0
(22) Date of filing: 20.11.2019
(51) Int. Cl.: C07D 233/84, A61P 1/00, A61P 11/00, A61K 31/4178

(54) **5-THIOISTIDINE COMPOUNDS AND METHYLATED DERIVATIVES (OVOTHIOLS) AS INHIBITORS OF GAMMA-GLUTAMYL-TRANSPEPTIDASE (GGT) ACTIVITY**
5-THIOISTIDIN-VERBINDUNGEN UND METHYLIERTE DERIVATE (OVOTHIOLE) ALS INHIBITOREN DER AKTIVITÄT DER GAMMA-GLUTAMYL-TRANSPEPTIDASE (GGT)
COMPOSÉS DE 5-THIOISTIDINE ET DÉRIVÉS MÉTHYLÉS (OVOTHIOLS) EN TANT QU'INHIBITEURS DE L'ACTIVITÉ DE GAMMA-GLUTAMYL-TRANSPÉPTIDASE (GGT)

(30) Priority: 10.12.2018 IT 201800010907
(43) Date of publication of application: 17.06.2020
(73) Proprietor: Stazione Zoologica "Anton Dohrn", 80121 Napoli (IT)
(72) Inventor: CASTELLANO, Immacolata, 80121 Napoli (IT); PALUMBO, Anna, 80121 Napoli (IT); D ARGENIO, Giuseppe, 80121 Napoli (IT)
(74) Representative: Currado, Luisa

(56) References cited:
- WO-A1-02/28869
- WO-A1-2018/144718
- WO-A1-2019/058247
- ASIYE KANBAY ET AL: "Serum gamma-glutamyl transferase activity is an independent predictor for cardiovascular disease in Obstructive Sleep Apnea Syndrome", RESPIRATORY MEDICINE, BAILLIERE TINDALL, LONDON, GB, vol. 105, no. 4, 2 December 2010 (2010-12-02), pages 637-642, XP028154216, ISSN: 0954-6111, DOI: 10.1016/J.RMED.2010.12.003 [retrieved on 2010-12-07]

## Description

### Background of the invention

The present invention relates to the field of chemistry and to the pharmaceutical sector since it concerns a class of inhibitors of gamma-glutamyl transpeptidase GGT, 5-thiohistidines and their methylated derivatives and their use for the prevention and the treatment of GGT-dependent pathologies as well as their use in GGT activity assays.

### State of the art

Thiohistidines are a class of sulfur-containing amino acids with key properties in the removal of peroxides (Holler, T.P. and Hopkins, P.B., 1988, Ovothiols as biological antioxidants. The thiol groups of ovothiol and glutathione are chemically distinct. J. Am. Chem. Soc., 110, 4837-4838; Shapiro, B.M., Turner, E.E., Hopkins, P.B., Klevit, R.E., Holler, T.P., Spaltenstein, A., 1990, Patent US 4898878 A "Antioxidant Thiohistidine Compounds"; Shapiro, B.M. and Hopkins, P.B., 1991, Ovothiols: biological and chemical perspectives, in Advances in Enzymology and Related Areas of Molecular Biology, ed A. Meister, 64, 291-316; Bailly, F., Vezin, H., Teissier, E., Duriez, P., Fruchart, J.C., Catteau, J.P., Bernier, J.L., 2000, 4-Mercaptoimidazoles derived from the naturally occurring antioxidant ovothiols 1. Antioxidant properties. Free Radic. Res., 32, 515-524).

Among these, 2-thiohistidines called ergothioneine are present mainly in some fungi and bacteria (Servillo, L., D'Onofrio, N., Casale, R., Cautela, D., Giovane, A., Castaldo, D., Balestrieri, M.L., 2017, Ergothioneine products derived by superoxide oxidation in endothelial cells exposed to high-glucose. Free Radic. Biol. Med.,108,8-18), whereas 5-thiohistidines are present mainly in marine invertebrates, bacteria and microalgae (Castellano, I., Migliaccio, O., D'Aniello, S., Merlino, A., Napolitano, A., Palumbo, A., 2016, Shedding light on ovothiol biosynthesis in marine metazoans. Sci. Rep., 6, 21506). These natural sulfur-containing products occur both as free amino acids and as constituents of an iron chelating pigment (Ito, S., Nardi, G., Palumbo, A., Prota, G., 1979, Isolation and characterization of adenochrome, a unique iron(III)-binding peptide from Octopus vulgaris. J. Chem. Soc. Perkin Transaction I, 2617-2623) or an alkaloid (Pathirana, C. and Andersen, R.J., 1986, Imbricatine, an unusual benzyltetrahydroisoquinoline alkaloid isolated from the starfish Dermasterias imbricata. J. Am. Chem. Soc., 108, 8288-8289). The 5-thiohistidines, thanks to the particular position of the thiol group on the imidazole ring of histidine, are probably the most acidic thiols among natural products and are also endowed with unique redox properties (Holler, T.P. and Hopkins, P.B., 1988, Ovothiols as biological antioxidants. The thiol groups of ovothiol and glutathione are chemically distinct. J. Am. Chem. Soc., 110, 4837-4838; Shapiro, B.M. and Turner, E., 1988, Oxidative stress and the role of novel thiol compounds at fertilization. Biofactors, 1, 85-88; Jacob, C., 2006, A scent of therapy: pharmacological implications of natural products containing redox-active sulphur atoms. Nat. Prod. Rep., 23, 851-863). In particular, ovothiols, methylated 5-thiohistidines, can play a key role in controlling cellular redox balance, thanks to their ability to carry out redox exchange with glutathione. In particular, ovothiol A is a methyl-5-thiohistidine, isolated and characterized from the eggs of the sea urchin *Paracentrotus lividus* (Palumbo, A., d'Ischia, M., Misuraca, G., Prota, G., 1982, Isolation and structure of a new sulphur containing aminoacid from sea urchin eggs. Tetrahedron Lett., 23, 3207-3208) and other marine invertebrates (Palumbo, A., Misuraca. G., d'Ischia, M., Donaudy, F., Prota, G.,1984, Isolation and distribution of 1-methyl-5-thiol-L-histidine disulphide and a related metabolite in eggs from echinoderms. Comp. Biochem. Physiol., 78B, 81-83). Ovothiol A has also been recently identified in the green microalgae *Euglena gracilis,* which is known for its potential in biotechnological applications, thanks to the easy cultivation and the high diversity of the metabolic profile (O'Neill, E.C., Trick, M., Hill, L., Rejzek, M., Dusi, R.G., Hamilton, C.J., Zimba, P.V., Henrissat, B., Field, R.A., 2015, The transcriptome of Euglena gracilis reveals unexpected metabolic capabilities for carbohydrate and natural product biochemistry. Mol. Biosyst., 11, 2808-2820).

Ovothiol A, isolated from sea urchin eggs, is already known to induce the arrest of cell proliferation through an autophagic mechanism in hepatocellular carcinoma cell lines (Russo, G.L., Russo, M., Castellano, I., Napolitano, A., Palumbo, A. (2014) Ovothiol isolated from sea urchin oocytes induces autophagy in the Hep-G2 cell line. Mar. Drugs, 12, 4069-4085) and to exhibit anti-inflammatory activity in an *in vitro* model of endothelial dysfunction associated with diabetic pathology, as described in the Italian patent application. N. 102017000104529 and International patent PCT, N. PCT/IB2018/057098.

The gamma-glutamyl transpeptidase enzyme (GGT, EC 2.3.2.2) is a glycosylated protein located on the cell surface that cleaves extracellular gamma-glutamyl compounds including glutathione and its oxidized and reduced S-conjugates (Hanigan MH, 2014, Gamma-glutamyl transpeptidase: redox regulation and drug resistance. Advan Cancer Res 122:103-141). In particular, GGT catalyzes the splitting of γ-glutamyl-donor substrates and the transfer of the γ-glutamyl portion to an amine of an acceptor substrate or to water. Thus, GGT plays a key role in the metabolism of glutathione and in the transport of amino acids into and out of the cell. Thanks to its ability to metabolize glutathione, GGT is involved in the detoxification processes and in the cellular response to oxidative stress and plays a key role in the evolution of many physiological disorders, such as tumor progression, chemotherapy drug resistance and neurodegenerative diseases (Castellano I & Merlino A, 2012, γ-Glutamyltranspeptidases: sequence, structure, biochemical properties, and biotechnological applications. Cell. Mol. Life Sci. doi 10.1007/s00018-012-0988-3. Review).

Several human pathologies are associated with the high expression and/or activity of GGT. For example in some tumors of the kidney it is known that the high activity of GGT induces the continuous metabolism of extracellular glutathione which results in a rapid recovery of the intracellular cysteine, necessary for the new protein synthesis and the rapid cell division, which thus contribute to tumor proliferation, or in any case resistance to chemotherapy drugs (Hanigan MH, Gallagher BC, Townsend DM, Gabarra V, 1999, Gamma-glutamyl transpeptidase accelerates tumor growth and increases the resistance of tumors to cisplatin in vivo. Carcinogenesis 20:553-559). Furthermore, it is known that high GGT expression/activity contributes to the pathology of asthma and renal ischemia/reperfusion injury (Yamamoto S, Watanabe B, Hiratake J, Tanaka R, Ohkita M, Matsumura Y, 2011, Preventive effect of ggstop, a novel and selective gamma-glutamyl transpeptidase inhibitor, on ischemia/reperfusion-induced renal injury in rats. J Pharmacol Exp Ther 339:945- 951, 3; Tuzova M, Jean JC, Hughey RP, Brown LA, Cruikshank WW, Hiratake J, Joyce-Brady M, 2014, Inhibiting lung lining fluid glutathione metabolism with ggstop as a novel treatment for asthma. Front Pharmacol 5:179). For example, the loss of GGT activity of the lung lining fluid in the mutant GGT mouse (enu1) causes an increase in the glutathione content of the lining fluid of the lung. The increase in glutathione is protective against asthma and in general against inflammation of the allergic airways. Therefore, normal mice are susceptible to asthma but can be protected by inhibitors of GGT activity (Tuzova M, Jean JC, Hughey RP, Brown LA, Cruikshank WW, Hiratake J, Joyce-Brady M, 2014, Inhibiting lung lining fluid glutathione metabolism with ggstop as a novel treatment for asthma. Front Pharmacol 5:179). Similarly, treatment with GGT inhibitors attenuates ischemia/ reperfusion-induced renal dysfunction in a dose-dependent manner in rats (Yamamoto S, Watanabe B, Hiratake J, Tanaka R, Ohkita M, Matsumura Y, 2011, Preventive effect of ggstop, a novel and selective gamma-glutamyl transpeptidase inhibitor, on ischemia/reperfusion-induced renal injury in rats. J Pharmacol Exp Ther 339:945- 951, 3). Therefore, in recent years, intense efforts have been devoted to the identification of inhibitors of the GGT enzyme due to their therapeutic potential in the treatment of asthma, renal ischemia/ reperfusion injury and chemo-resistance to some types of cancer. However, several GGT inhibitors identified in the past, such as acivicin, have been abandoned in clinical studies for their toxicity (King JB, West MB, Cook PF, Hanigan MH (2009) A novel, species-specific class of uncompetitive inhibitors of gamma-glutamyl transpeptidase. J Biol Chem. 284:9059-65. doi: 10.1074/jbc.M809608200). For example, the glutamate analogue, 6-diazo-5-oxo norleucine (DON), in addition to inhibiting GGT, also inhibits many enzymes that metabolize glutamine, making it too toxic for clinical use as GGT inhibitor (Terzyan SS, Burgett AW, Heroux A, Smith CA, Mooers BH, Hanigan MH, 2015, Human gamma-glutamyl transpeptidase 1: structures of the free enzyme, inhibitor-bound tetrahedral transition states, and glutamate-bound enzyme reveal novel movement within the active site during catalysis. J Biol Chem 290:17576-17586). Previous studies have shown that DON is an irreversible inhibitor of human GGT (hGGT1) and this makes it not very versatile. Subsequently an inhibitor that binds the acceptor site, OU749, more specific and less toxic than the glutamine analogues, was identified. (King JB, West MB, Cook PF, Hanigan MH (2009) A novel, species-specific class of uncompetitive inhibitors of gamma-glutamyl transpeptidase. J Biol Chem. 284:9059-65. doi: 10.1074/jbc.M809608200). However, the inhibitors known so far, including the analogues of the physiological substrate, glutathione, have been found to be toxic and/ or irreversible (Nakajima M, Watanabe B, Han L, Shimizu B, Wada K, Fukuyama K, Suzuki H, Hiratake J, 2014, Glutathione-analogous peptidyl phosphorus esters as mechanism-based inhibitors of γ-glutamyl transpeptidase for probing cysteinyl-glycine binding site. Bioorg Med Chem. 22:1176-94. doi: 10.1016/j.bmc.2013., Kathryn M. Lemberg, James J. Vornov, Rana Rais and Barbara S. Slusher. We're Not "DON" Yet: Optimal Dosing and Prodrug Delivery of L-6-Diazo-5-oxo-L-norleucine. DOI: 10.1158/1535-7163.MCT-17-1148).

Liver fibrosis is a complex process caused by chronic liver damage, which leads to an excessive increase in the accumulation of extracellular matrix proteins and fibrogenesis. Liver tissue is characterized by high levels of GGT and chronic inflammation of the liver can cause damage to the membranes resulting in release of GGT in the blood (Yu, Y., Fan, Y., Yang, Z., Lu, Y., Xu, Q., Chen, X., 2016, Elevated serum gamma-glutamyltransferase predicts advanced histological liver damage in chronic hepatitis B. Discov. Med. 21, 7-14). The increase of GGT activity in serum was in fact widely used as an index of liver dysfunction and marker of excessive alcohol intake (Whitfield, J.B., 2001, Gamma glutamyl transferase. Crit. Rev. Clin. Lab. Sci. 38, 263-355).

A neuroprotective effect of a modified synthetic analogue of ovothiol, 1-methyl-2- (3-trifluoromethylphenyl) -4-mercaptoimidazole is known (MFP-4MI) (Vamecq, J., Maurois, P., Bac, P., Bailly, F., Bernier, J.L., Stables, J.P., Husson, I., Gressens, P. (2003) Potent mammalian cerebroprotection and neuronal cell death inhibition are afforded by a synthetic antioxidant analogue of marine invertebrate cell protectant ovothiols. Eur. J. Neurosci., 18, 1110-1120).

The 5-methyl-thiohistidine, ovothiol A, is able to induce an autophagic process on liver tumor cell lines, through the activation of autophagy markers such as LC3 and beclin-1 (Russo, G.L., Russo, M., Castellano, I., Napolitano, A., Palumbo, A. (2014) Ovothiol isolated from sea urchin oocytes induces autophagy in the Hep-G2 cell line. Mar. Drugs, 12, 4069-4085).

The anti-inflammatory and anti-atherogenic effect of ovothiol A on endothelial dysfunction associated with cardiovascular diseases, such as diabetes, has been further described. In this case the ovothiol induces an increase in the bioavailability of Nitric Oxide which induces a reduction in the adhesion of monocytes to the endothelium, thus mimicking the inhibition of the atherosclerotic process (Castellano I, Di Tomo P, Di Pietro N, Mandatori D, Pipino C, Formoso G, Napolitano A, Palumbo A, Pandolfi A. (2018) "Anti-inflammatory activity of marine ovothiol A in an in vitro model of endothelial dysfunction induced by hyperglycemia" Oxid Med Cell Longev, 2087373. doi.org/10.1155/2018/2087373) .

The Italian patent application No. 102017000104529 and the International Patent Application No. PCT/IB2018/057098 describe the use of ovothiols for the prevention and treatment of low-grade chronic systemic inflammation (ISC) and of ISC-related diseases that include: chronic inflammatory diseases, autoimmune diseases, chronic degenerative diseases, ISC-related tumors, obesity, diabetes, arterial hypertension, hypercholesterolemia, cardio-vascular diseases, osteoporosis, respiratory diseases and polycystic ovary syndrome.

The US Patent n. US 4898878 describes active ovothiol derivatives in which the substituents are individually selected from hydrogen, methyl or other atoms and groups that do not negatively influence the overall redox activity spectrum of the molecule.

The US Patent Application n. 20100168198 describes a pro-antioxidant drug targeted to mitochondria, useful for the prevention or treatment of diseases or conditions associated with mitochondrial dysfunction resulting from changes in the mitochondrial redox environment. The pro-drugs are produced by modifying an antioxidant in a fatty acid so that the resulting pro-drug is targeted and activated by an enzyme responsible for the beta-oxidation of mitochondrial fatty acids. The examples describe the activation of beta-oxidation of fatty acids of the anti-oxidant pro-drugs based on 4-mercaptoimidazole (ovothiol).

Different classes of compounds with inhibitor effect on enzymes of the GGT family are described in the international patent applications n. WO2014JP72555 and WO0228869 and in the US patent applications n. US2010197745 and n. US2014024685.

### Closest Prior Art

Holler et al., 1989, Total synthesis of marine mercaptohistidines: Ovothiols A,B and C, J. Organic Chem., 54(19). 4570-4575 describes a racemic mixture of ovothiol A.

Nathchar Naowarojna et al., 2018, In vitro reconstitution of Remaining Steps in Ovothiol A Biosynthesis: C-S Lyase and Methyltransferase Reactions" Organic Letters, 20(17), 5427-5430 describes two of the enzymatic activities responsible for the biosynthesis of the ovothiol A.

Gian Luigi Russo et al., 2014, Ovothiol isolated from Sea Urchin Oocytes Induces Autophagy in Hep-G2 cell line, 12(7), 4069-4085, describes the use of ovothiol A disulfide in the treatment of hepatocellular carcinoma cells.

The international patent application, publication number WO 2018/144718, describes the prodrugs of acivicin, an inhibitor of y-glutamyl transferase.

The international patent application, publication number WO 02/28869 describes some derivatives of glutamine as y-glutamyl transpeptidase inhibitors.

Asiye Kanbay et al, 2011, Serum gamma-glutamyl transferase activity is an independent predictor for cardiovascular disease in Obstructive Sleep Apnea Syndrome, Respiratory Medicine, 105, 637-642 discloses reports that GGT was identified as a marker of low-grade systemic inflammation.

### Technical Problem

The compounds of natural origin to be used for the design of drugs in order to overcome the side effects of chemical synthesis products are of considerable interest in the biochemistry and pharmaceutical sector.

For this reason, enormous efforts are devoted to the discovery of new active compounds from natural sources, such as the marine ones, characterized by a greater chemical biodiversity compared to the terrestrial ones.

Furthermore, the identification and characterization of new GGT inhibitors for the prevention and treatment of physiological disorders linked to high levels of GGT are of considerable interest.

The GGT inhibitors of chemical synthesis known so far have several drawbacks: for the most part they are irreversible GGT inhibitors whose effects are difficult to modulate, are toxic and are characterized by a low specificity of action.

In light of the state of the art, the inventors of the present invention have investigated the inhibitory effect of the class of 5-thioistidine compounds , including the methylated analogues, in their disulphide form, on the activity of GGT enzymes.

The same inventors of the present invention have verified that these compounds act as non-competitive inhibitors of GGT, and show an inhibition power of 10-20 times higher than that of other commercial inhibitors such as DON and are not toxic at the recommended doses.

Furthermore, the inventors have verified the potential efficacy in the treatment of diseases related to high production or GGT activity such as, for example, in the regression of the liver fibrosis process. In fact, they have shown that during the process of hepatic fibrosis, the activity of the mature GGT form anchored to the membrane increases, probably contributing to membrane damage, which in turn causes the release of the protein itself in the serum. Treatment with ovothiol in mice affected by the fibrotic process causes the reversion of this phenomenon, i.e. a reduction in membrane GGT activity at the physiological levels of healthy mice, most likely contributing to maintain membrane integrity and reduce its release into the serum.

### Object of the invention

With reference to the attached claims, the technical problem is therefore solved by providing the compounds of formula: 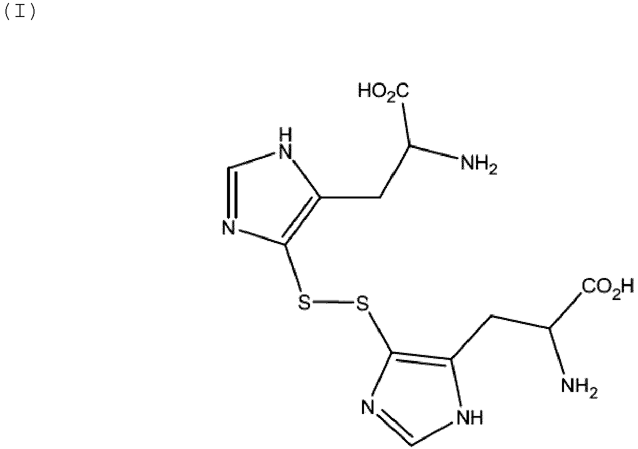 and 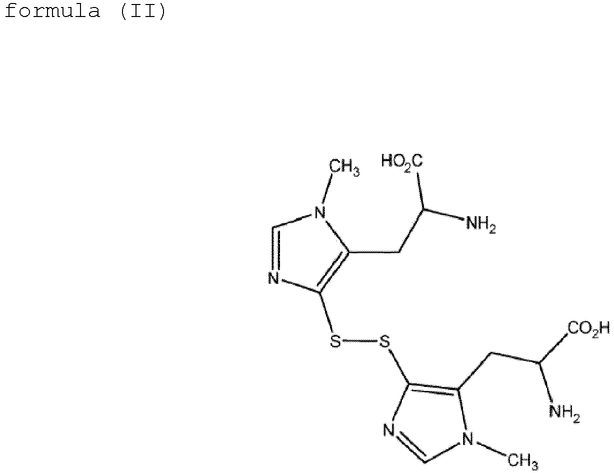 for use in the prevention and the treatment of renal ischemia/reperfusion damage, heart failure, cholestasis, pancreatitis, hepatopathies, chronic obstructive pulmonary disease (COPD), respiratory tract infections, pathologies of the upper respiratory tract, cirrhosis of the liver, hepatic ischemia, necrosis of the liver, hepatitis, steatosis, hepatotoxic drug intoxication, hepatic fibrosis.

### Brief description of the drawings

Figure 1 shows in graph the effect of 5-thiohistidine (5-thio), ovothiol A, and ergotioneine (N-alpha-trimethyl-2-thiohistidine) (Erg) on the GGT activity.
Figure 2 shows in graph the kinetics of inhibition of 5-thio, ovothiol A, Erg and DON carried out by varying the concentration of the acceptor substrate Gly-Gly (glycyl-glycine).

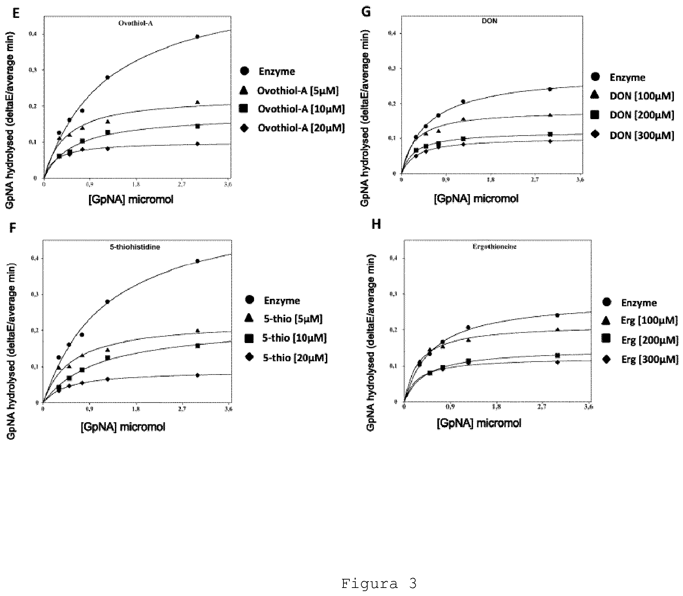

Figure 3 shows in graph the kinetics of inhibition of 5-thio, ovothiol A, Erg and DON carried out by varying the concentration of the donor substrate GpNA.

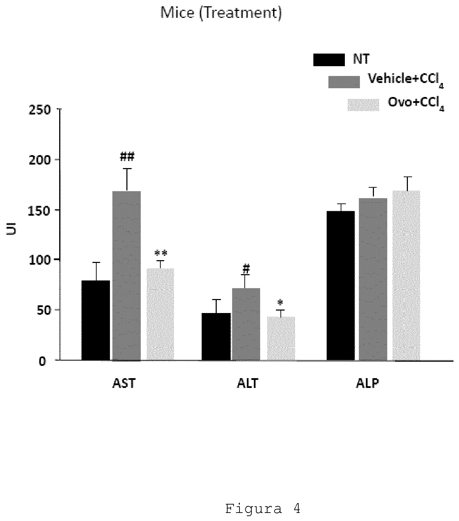

Figure 4 shows the measurement, through standard laboratory assays, of the levels of the enzymes responsible for liver function, aspartic-transferase (AST), alanine-transferase (ALT), alkaline-phosphatase (ALP) in the serum of mice.

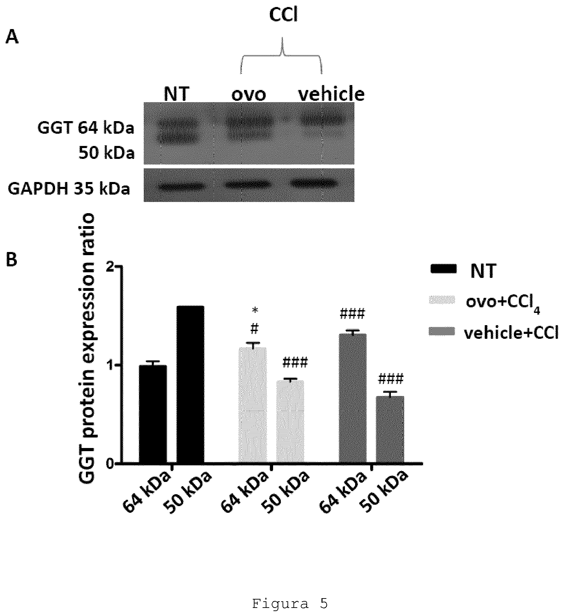

Figure 5 shows in panel A) the Western Blot carried out using an antibody specific for GGT in microsomal extracts obtained from liver tissue of mice in which hepatic fibrosis was induced and treated with ovothiol or with control solution, compared to healthy mice untreated (NT); in panel B) the histogram of the densitometric analysis of the 64 and 50 kDa bands, normalized for GAPDH; the bars indicate the average of the measurements from 7 different individuals +/- SD (standard deviation); the statistical significance determined by the one-way ANOVA test, # (p <0.05), and ### (p <0.001) significance with respect to healthy controls; * (p <0.05), *** (p <0.001) significance compared to fibrotics treated with control solution.

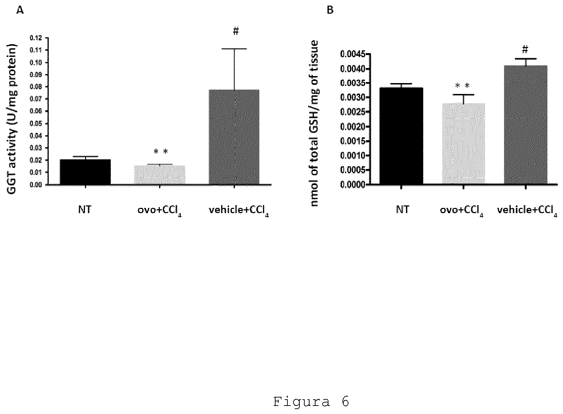

Figure 6 shows in panel A) the enzymatic activity of GGT evaluated by spectrophotometric assay with a chromogenic GpNA substrate on microsomal extracts of liver tissue containing GGT bound to the membrane, and the activity of GGT is normalized with respect to the mature protein band (50 kDa) highlighted by western blot; in panel B) the levels of intracellular thiols in fibrotic liver tissues treated with ovothiol A or with control solution determined by Glutathione Assay Kit; the bars indicate the average of 7 measurements +/- SD (standard deviation), the significance was determined by the Student test, # (p <0.05) significance with respect to the healthy control; ** (p <0.01) significance compared to fibrotic mice treated with control solution.

### Detailed description of the invention

### Definitions

Within the meaning of the present invention, GGT gamma-glutamyl transpeptidase inhibitor means any compound able to inhibit the activity of the gamma-glutamyl transpeptidase enzyme GGT, i.e. the catalysis of the cleavage reaction of γ-glutamyl- substrates donors and the transfer of the γ-glutamyl portion to an amine of an acceptor substrate or to water. This enzyme is involved in the metabolism of glutathione and in the cellular transport of amino acids.

Within the meaning of the present invention, GGT-related pathologies means the pathologies known to be sensitive to the use of GGT inhibitors such as renal damage due to ischemia /reperfusion, diseases associated with reverse airway obstruction such as asthma, chronic obstructive pulmonary disease (COPD), allergic reaction, respiratory tract infection or upper respiratory tract disease; pathologies related to increased levels of GGT activity in the blood such as heart failure, cholestasis (biliary tract congestion), pancreatitis, liver diseases such as liver cirrhosis, hepatic ischemia, liver necrosis, hepatitis, steatosis, hepatotoxic drug intoxication, liver fibrosis; excluding diseases related to ISC Chronic systemic inflammation (ISC).

The present invention concerns the compounds of formula: 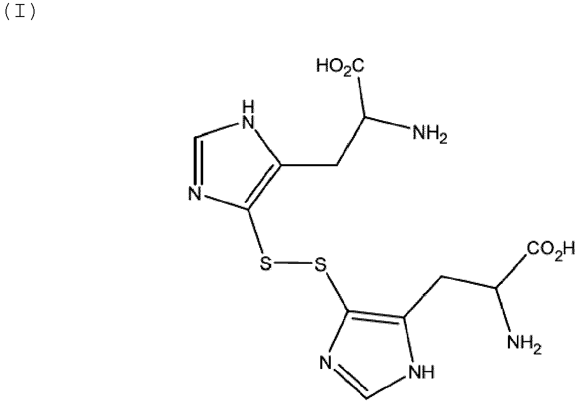 And 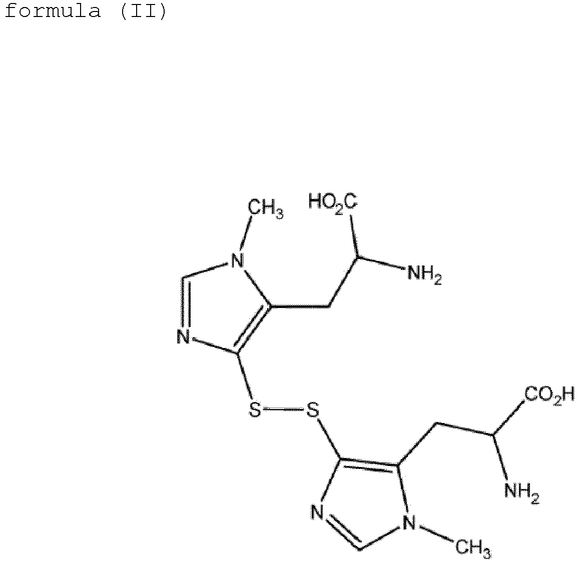 for use in the prevention and the treatment of renal ischemia/reperfusion damage, heart failure, cholestasis, pancreatitis, hepatopathies, chronic obstructive pulmonary disease (COPD), respiratory tract infections, pathologies of the upper respiratory tract, cirrhosis of the liver, hepatic ischemia, necrosis of the liver, hepatitis, steatosis, hepatotoxic drug intoxication, hepatic fibrosis.

### Examples

### METHODS

Production and purification of 5-thiohistidines and methylated derivatives (ovothiols): the non-methylated 5-thiohistidine (5-thio) was supplied by Prof. Florian P. Seebeck of the University of Basel in Switzerland and was prepared by chemical synthesis according to the protocol described in Daunay S., Lebel R., Farescour L., Yadan J.C. and Erdelmeier I. (2016) "Short protecting-group-free synthesis of 5-acetylsulfanyl-histidines in water: novel precursors of 5-sulfanyl-histidine and its analogues". Org Biomol Chem, 14 (44): 10473-10480. Ovotiol A was extracted and purified from the sea urchin eggs *Paracentrotus lividus* according to the protocol described by Russo, G.L., Russo, M., Castellano, I., Napolitano, A., Palumbo, A. (2014) Ovothiol isolated from sea urchin oocytes induces autophagy in the Hep-G2 cell line. Mar. Drugs, 12, 4069-4085. Both 5-thio and ovotiol A were used in the form of disulfide because the reduced form is very reactive.

Induction of experimental hepatic fibrosis: 19 mice (weight 25-30g), kept in the enclosure of the Department of Clinical Medicine and Surgery of the University of Naples Federico II, were used to study the hepatic fibrosis process. Mice housed in a room, with a constant average temperature of 22 ° C, with a 12-hour lighting cycle, had free access to food and water. In 14 mice hepatic fibrosis was induced by intraperitoneal injections of carbon tetrachloride (CCl4) 0.2 mL / 100 g of body weight twice a week, for 7 weeks. The mice, divided into two groups of 7, were treated as follows: with intraperitoneal injection of ovothiol A in the form of disulfide in aqueous solution (50 µg / kg) 3 times a week for 7 weeks (first group) or intraperitoneal injection of the aqueous vehicle only 3 times a week for 7 weeks (control group). A further group of 5 healthy mice were included in the study. Repeated cycles of hepatic inflammation and repair damage cause fibrosis. The optimal timing of the sacrifice has been established on the basis of previous experience and on data from articles showing that spontaneous recovery of liver lesions is a very slow process. At the end of the treatment the mice were sacrificed by cardiac puncture; a blood sample was taken, then the liver was removed from which various samples were obtained for subsequent experimental investigations. The liver tissues were frozen at a temperature of -80 ° C for subsequent molecular analyses.

Histological analysis: a sample of hepatic tissue, fixed in formalin and included in paraffin was cut into 4µm sections with the microtome. The representative sections of each hepatic sample were stained with hematoxylin/eosin according to standard protocols. All histological analyses were performed by an expert in histopathology of the Department of Clinical Medicine and Surgery of the University of Naples Federico II. For collagen detection and quantification, the sections were colored with the Sirius red / Fast green solution. The extent of liver fibrosis was determined as the percentage of area of each section, highlighted with the dye. A Sony 3CCD videomicroscope (model DXC-950P) equipped with a motor stage and the Quantimed 500MC software (Leica, Germany) was used to analyze the morphological structure.

Isolation of GGT from tissues or cells: the murine liver tissues were homogenized with a Potter at 4 ° C in 5 volumes of Tris-HCL 25 mM, pH 7.5, EDTA 0.2 mM, containing sucrose 0.33 M, leupeptin 1 µM and 1.4 µg/ml aprotinin. The homogenate was centrifuged at 9000 x g for 20 minutes, the supernatant was centrifuged at 100,000 x g for 1 hour to precipitate nuclei, mitochondria and other cellular organelles. The pellet was homogenized in Tris-HCL 25 mM, pH 7.35, Triton X-100 0.5%, leupeptin 1 µM, 1.4 µg/ml aprotinin and then centrifuged again at 100000 x g for 1 hour. The supernatant was analysed for GGT protein activity or expression, aliquoted and stored at -80° C until the next use.

Protein expression of GGT: the microsomal liver extracts (about 10 µg) were analyzed in electrophoresis on 12% polyacrylamide SDS gel in Laemmli buffer. Following electrophoresis, the proteins were transferred to a PVDF membrane (Millipore) (Biorad transblot apparatus) and detected using a mouse anti-GGT monoclonal antibody (Santa Cruz Biotechnology, USA) and as an internal control a monoclonal murine antibody anti-GAPDH (Santa Cruz Biotechnology, USA). The primary antibody was incubated 1: 2000 in PBS 1X 4% milk at 4 ° C overnight, after incubation with 5% milk in 1X PBS. The appropriate secondary antibody was added and the immunoreactive proteins were detected using the ECL (Western Bright TM detection kit ECL, Advansta, USA). Protein expression levels were analyzed by densitometric analysis using the Image J. software.

GGT activity assay: GGT activity was determined by a colorimetric test. The analysis buffer contains 100 mM Tris-HCl pH 7.8 or PBS 1X pH 7.4. Each reaction contains 1 mM of γ-glutamyl-para-nitroanilide (GpNA) as a donor substrate and 40 mM glycyl-glycine (glygly) as an acceptor substrate. The enzyme GGT purified from murine tissues or human cells, or from equine sources (purchased from Sigma) was added at the end to start the test. The formation of the product, p-nitroaniline, was continuously monitored at room temperature at 405 nm using a Bio-Rad 680 microplate reader with the Microplate Manager 5.2 software (Bio-Rad). One unit of GGT activity was defined as the amount of GGT that released 1 µmοl of p-nitroaniline/min at room temperature. The molecules tested as inhibitors, Erg, DON and DTT were purchased by Sigma Aldrich.

Glutathione assay: total glutathione levels were determined by Glutathione Assay Kit (Sigma). Briefly, frozen liver tissues were crushed in a mortar with pestle in the presence of liquid nitrogen to prepare a fine powder. Then 100 mg of powder were added to 3 volumes of 5% sulfosalicylic acid and mixed. Subsequently, another 7 volumes of 5% 5-sulfosalicylic acid were added and mixed for 5 minutes at 4 ° C and finally centrifuged at 10,000 g for 10 minutes. Diluted samples were used for the analysis procedure, in which, after incubation with glutathione reductase and NADPH, glutathione was completely recovered in reduced form and then determined by monitoring the reduction of 5,5-dithiobis (2-nitrobenzoic acid) (DTNB) at 5-thio-2-nitrobenzoic (TNB), at 412 nm using a Thermo Scientific ^{™} Multiskan ^{™} FC microplate spectrophotometer.

### Example 1: effect of 5-thio, ovothiol A, and Erg on GGT activity.

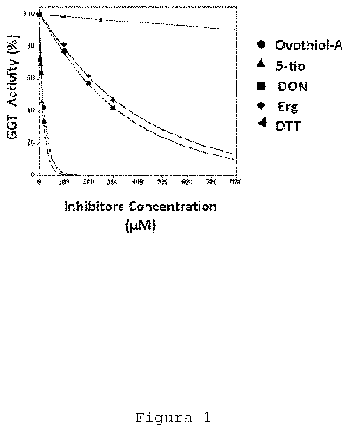

The enzymatic activity assays were carried out using the Tate & Meister spectrophotometric assay, using the γ-glutamyl-para-nitroanilide chromogen (GpNA) as a donor substrate, glycyl-glycine as a substrate and a purified commercial GGT solution as a biocatalyst from horse (EqGGT) with a high % identity with the human one. These assays were also performed in the presence of GGT isolated directly from the hepatic mouse tissues and/or from human cell cultures (hGGT). The inhibition of EqGGT and hGGT by 5-thio and ovothiol A was compared with that of Erg and the GGT DON commercial inhibitor. As a negative control, a non-specific thiol, dithiothreitol (DTT) was used, and the results are shown in figure 1. The concentration at which these compounds induce 50% inhibition of GGT activity (IC50) are shown in Table 1 below. The ovothiol A and 5-thio in their disulfide forms were found to be the most active compounds in inhibiting GGT activity. In particular they are 10-20 times more active than Erg (2-thioistidine stable in its thionic form) and DON, a less specific GGT inhibitor abandoned in clinical trials for toxicity. Comparable values were obtained using hGGT isolated from human HG3 cell lines, over-expressing GGT. Both the ovothiol and the non-methylated 5-thiohistidine form show no cell toxicity up to concentrations of 200 µM in human cell lines, such as renal embryonic origin HEK293 and keratinocytes HaCat. Instead, the already known GGT inhibitors are toxic at much lower concentrations.

**Table 1**

| **Compound** | **IC₅₀** |
|---|---|
| Ovothiol A | 16 µM |
| 5-thio | 13 µM |
| DON | 250 µM |
| Erg | 277 µM |
| DTT | ND |

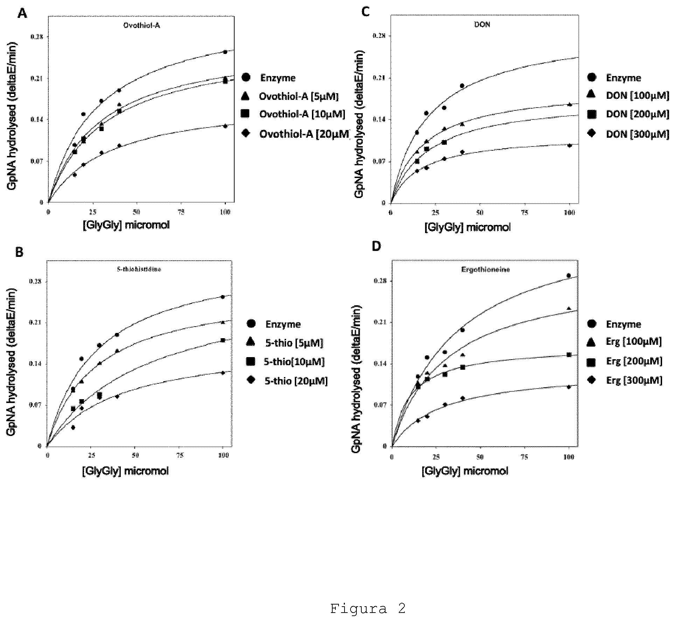

The inhibition constants Ki and GGT inactivation rate for 5-thio and ovothiol A were determined by varying both the concentrations of the GpNA donor substrate and maintaining the Gly-Gly acceptor concentrations fixed and vice versa, as shown in Figure 2. Ki (dissociation constant related to E) was calculated as a function of Vmax. For all the compounds the Vmax decreases as the concentration of inhibitor increases, while the km remains almost unchanged. These data suggest that 5-thio, ovothiol A and Erg behave as non-competitive inhibitors both with respect to the acceptor substrate and to the donor substrate and confirm that the compounds belonging to the class of 5-thioistidines are about 10-30 times more potent in inhibiting GGT compared to DON and Erg.

Table 2 shows the Ki values that represent the Ki averages obtained from the two methods used to derive the Vmax and Km, namely Michaelis-Menten and Lineweaver-Burk.

**Table 2**

| **Compound** | **Ki (gly-gly)** | **t-Probability** | **Ki (GpNA)** | **t-Probability** |
|---|---|---|---|---|
| Ovothiol-A | 26.1 ± 12.3 | 0.003 | 4.5 ± 1.96 | 0.002 |
| 5-thiohistidin | 17.4 ± 6.1 | 0.003 | 6.9 ± 2.91 | 0.002 |
| DON | 199.8 ± 62.7 | 0.002 | 156.5 ± 15.3 | <0.0001 |
| Ergothioneine | 240.6 ± 32,2 | <0.0001 | 228.8 ± 53.92 | 0.0002 |

### Example 2: Effect of ovothiol A in a mouse model of liver fibrosis associated with high levels of GGT

Liver fibrosis was induced in 2 different groups of mice by intraperitoneal injection of carbon tetrachloride. Only one of these groups was administered ovothiol A at a dose of 50 mg/kg 3 times a week for almost 2 months. Since both the methylated and non-methylated forms of 5-thiohistidines inhibit similarly the GGT activity, we have only used the first one for the example given. Histological analysis of the liver tissues of CCl4-treated mice showed the onset of the fibrotic process, as demonstrated by the increase in collagen fibers deposited in the extracellular matrix, compared to liver tissues from healthy mice. Treatment with ovothiol A, on the other hand, significantly reduced the progression of the fibrotic process, as observed by the reduction in the amount of collagen fibers deposited compared to mice with liver fibrosis and treated with control solution (2.7 + 0.9% vs 5.8 + 1.2%, p <0.05). At the same time, the determination of serum levels of liver function index enzymes showed that the levels of aspartic-transferase (AST) and alanine-transferase (ALT) were reduced in mice treated with ovothiol compared to those treated with the control alone, thus confirming reversion of the fibrotic process.

The presence of GGT in liver tissue was assessed by immunoblot. GGT is synthesized as a single 64 kDa polypeptide precursor, which then undergoes self-proteolysis to form the mature protein, composed of two subunits, being the largest 50 kDa. The antibody used is directed against a peptide contained in the major subunit, so it is able to recognize both the latter and the precursor. In microsomal extracts of healthy liver tissue, 62% of total GGT is present in mature form (corresponding to the 50 kDa band), while in fibrotic tissues the mature form is significantly reduced, i.e. it represents 36% of the total. Conversely, treatment with ovothiol induces an increase in the mature form present in membranes up to 44%. The data shown in Figure 5 suggest that probably the form of GGT released into the blood during the progression of liver fibrosis is the mature form, and that treatment with ovothiol inhibits its release into the serum, probably reducing the damage to the membrane. GGT activity was determined in liver microsomal extracts and normalized against the amount of mature protein, which represents the active form of the enzyme, compared to the inactive precursor polypeptide. GGT activity increases significantly in mouse tissues affected by fibrosis, compared to healthy mice, indicating that the disease is among those characterized by high levels of GGT activity. Treatment with ovothiol, on the other hand, induces a significant reduction in GGT activity, indicating that the molecule exerts its anti-fibrotic activity through inhibition of the enzyme, as shown in Figure 6. GGT catalyses the degradation of extracellular GSH and the transfer of the γ-glutamyl group to amino acids to promote their recycling within the cell, or to small molecules to promote liver detoxification. The cysteinyl-glycine, resulting from the hydrolysis reaction of GSH, is one of the most reactive thiol compounds capable of reducing oxygen by redox reaction of the iron ion, thus promoting the increase of reactive oxygen species (ROS) and the oxidative reactions. Therefore, high levels of GGT activity can induce oxidative stress in the cell, thus contributing to the damage and development of liver fibrosis. On the other hand, ovothiol, by inhibiting the activity of GGT, also reduces the amount of cysteinyl-glycine that re-enters the cell, thus avoiding the accumulation of ROS and oxidative damage. To support this hypothesis, the determination of the intracellular GSH, or in any case of the thiol content, has shown that this increases in untreated fibrotic tissues and decreases with the treatment of ovothiol A, confirming that the inhibition of the hydrolysis of extracellular GSH reduces the cysteine recycling in the cell, and the accumulation of cysteinyl-glycine, and therefore the excessive synthesis of intracellular thiols.

## Claims

1. Compound of formula: 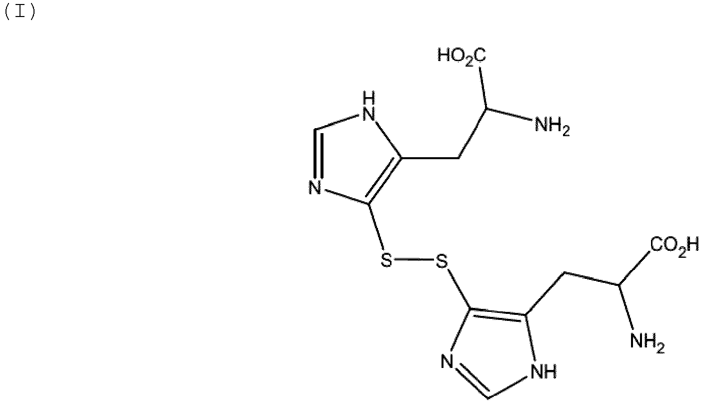 and 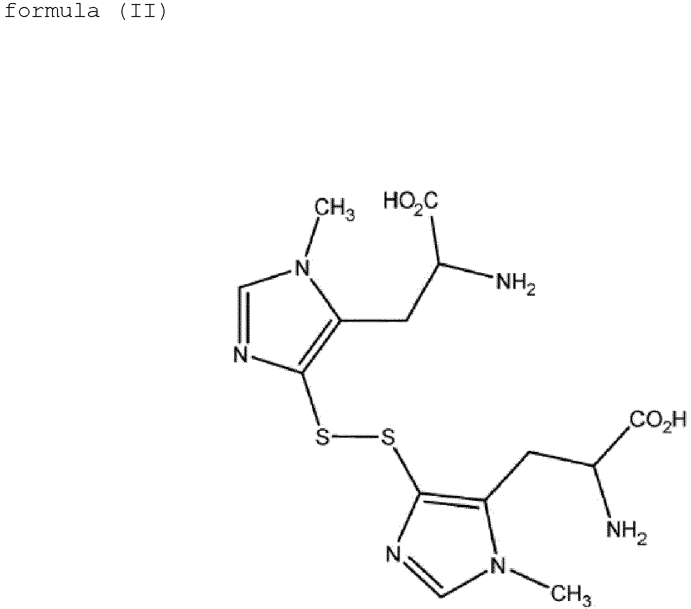 for use in the prevention and the treatment of renal ischemia/reperfusion damage, heart failure, cholestasis, pancreatitis, hepatopathies, chronic obstructive pulmonary disease (COPD), respiratory tract infections, pathologies of the upper respiratory tract, cirrhosis of the liver, hepatic ischemia, necrosis of the liver, hepatitis, steatosis, hepatotoxic drug intoxication, hepatic fibrosis.

## Patentansprüche

1. Verbindung der Formel: 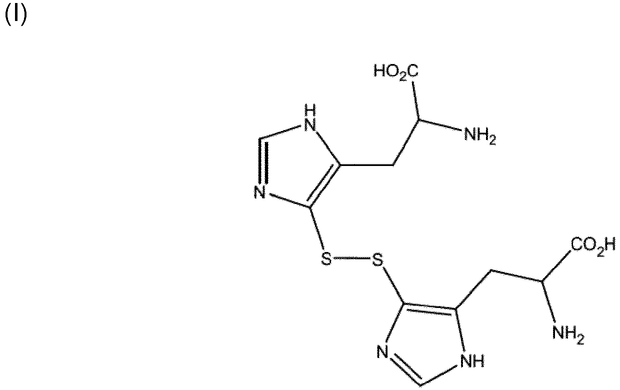 und 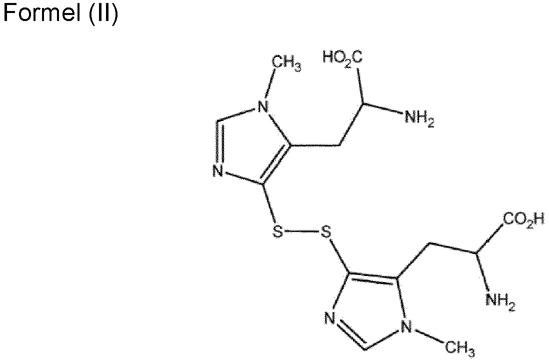 zur Verwendung bei der Vorbeugung und Behandlung von Nierenischämie/Reperfusionsschäden, Herzinsuffizienz, Cholestase, Pankreatitis, Hepatopathien, chronisch obstruktiver Lungenerkrankung (COPD), Infektionen der Atemwege, Erkrankungen der oberen Atemwege, Leberzirrhose, Leberischämie, Nekrosen der Leber, Hepatitis, Steatose, hepatotoxische Arzneimittelvergiftung, Leberfibrose.

## Revendications

1. Composé de formule: 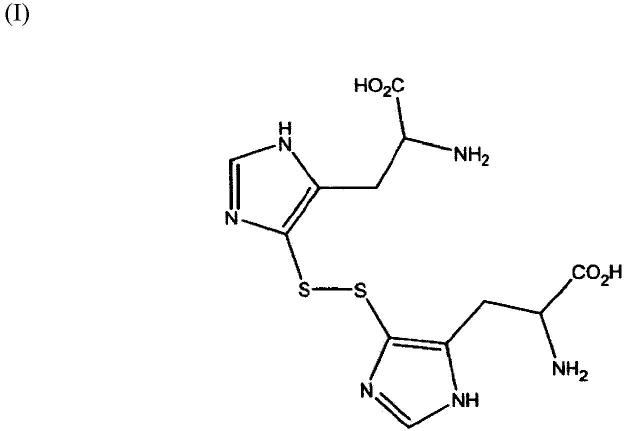 et de 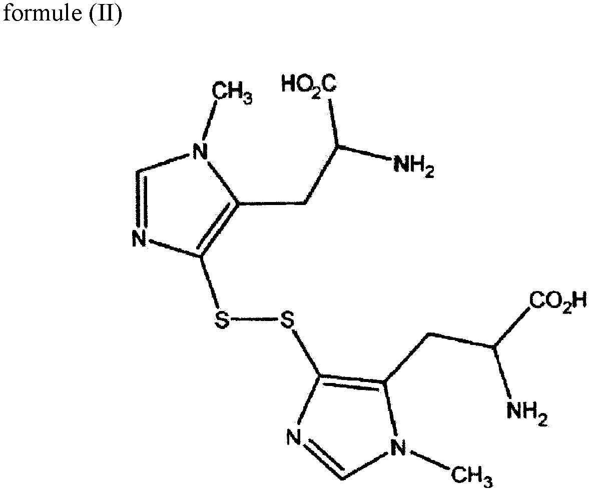 destiné à être utilisé dans la prévention et le traitement des lésions d'ischémie/reperfusion, de l'insuffisance cardiaque, de la cholestase, de la pancréatite, des hépatopathies, de la bronchopneumopathie chronique obstructive (BPCO), des infections des voies respiratoires, des pathologies des voies respiratoires supérieures, de la cirrhose du foie, de l'ischémie hépatique, de la nécrose du foie, de l'hépatite, de la stéatose, des intoxications par des médicaments hépatotoxiques, de la fibrose hépatique.
